# EUROPEAN PATENT SPECIFICATION

(11) **EP 4 524 529 B1**
(45) Date of publication and mention of the grant of the patent: **10.09.2025**
(21) Application number: 23820970.4
(22) Date of filing: 10.11.2023
(51) Int. Cl.: G01K 1/024, G01K 1/14, G01N 33/38

(54) **EQUIPMENT FOR MEASURING MOISTURE AND TEMPERATURE INSIDE THE WALLS AND THE SPACE WHERE THE MEASURING EQUIPMENT IS HOUSED**
VORRICHTUNG ZUR MESSUNG VON FEUCHTIGKEIT UND TEMPERATUR IN WÄNDEN UND RAUM, IN DEM DIE MESSVORRICHTUNG UNTERGEBRACHT IST
ÉQUIPEMENT DE MESURE DE L'HUMIDITÉ ET DE LA TEMPÉRATURE À L'INTÉRIEUR DES PAROIS ET DE L'ESPACE OÚ EST LOGÉ L'ÉQUIPEMENT DE MESURE

(30) Priority: 01.12.2022 ES 202231036
(43) Date of publication of application: 19.03.2025
(73) Proprietor: Martínez Lluch, Antonio, 46185 La Pobla de Vallbona Valencia (ES)
(72) Inventor: Martínez Lluch, Antonio, 46185 La Pobla de Vallbona Valencia (ES)
(74) Representative: Isern Patentes y Marcas S.L.
(86) International application number: PCT/ES2023/070673
(87) International publication number: WO 2024/115800

(56) References cited:
- EP-A1- 3 916 384
- US-A1- 2001 017 053
- US-A1- 2008 259 993
- US-A1- 2014 260 604

## Description

### Technical field

The present invention relates to electronic equipment for measuring and subsequently controlling moisture and temperature inside and/or outside a wall.

### State of the art

In the current state of the art, different moisture and temperature measuring devices applicable to walls are known. For example, document CN103389757A discloses a wall-mounted fully digital temperature and moisture meter comprising a casing, a display screen, a keyboard, a data exchange port, a temperature sensor, a moisture sensor, a temperature control interface, a moisture control interface and a central processing unit installed inside the casing. The wall-mounted fully digital temperature-moisture control meter further comprises an alarm apparatus. The signal input end of the alarm apparatus is connected to the alarm signal output end of the central processing unit. The alarm apparatus is installed in the position, near the front face of the casing, inside the casing. A sound transmission opening is formed in the position, corresponding to the alarm apparatus, of the front face of the casing. A wall-mounted device is provided on the rear face of the casing. Due to the fact that the wall-mounted device is arranged on the rear face of the casing, the wall-mounted fully digital temperature and moisture monitoring meter can be conveniently installed on the wall of rooms having high temperature requirements, such as hanging medicine storage, and it is convenient to use. Due to the fact that the alarm apparatus is installed and the sound of the alarm apparatus can be transmitted through the sound transmission opening, a manager can conveniently and promptly know whether or not the temperature and moisture exceed the ranges in order to make the right decision.

Document ES2608901A1 discloses an improved moisture sensor consisting of a capacitive moisture sensor, a temperature sensor, a frequency division multiplexer of the electrical signals from the moisture and temperature sensors and a microcomputer that digitises said electrical signals from the moisture and temperature sensors through an electrical connection compatible with RS485, the communications protocol of which is compatible with Modbus, and a heat generating source controlled by the aforementioned microcomputer. All of the foregoing components, together with the corresponding electrical and data wiring thereof, are arranged inside a hollow plastic cylinder, said cylinder having in one of its walls a through hole that communicates the inside of said cylinder with the outside environment, and said hole being closed by a water-impermeable and vapor-permeable membrane made from a mixture of cellulose, plastic and stainless steel.

Other publications similar to those described are documents ES2866626T3, CN204359739U and CN209841081A1. However, none of these documents disclose measuring equipment with the features of the equipment of claim 1 which accompanies the present specification.

Documents no. US 2014260604 and US 2008259993, which also describe equipment for measuring moisture and temperature, are also known in the state of the art. However, none of them have means envisaged to measure the internal temperature of the wall.

Also, it is known from prior art EP 3916384 A1 disclosing a measurement device intended to measure temperature and humidity, whose common features are part of the preamble of claim 1.

### Description of the invention

The object of the present invention is to provide a novel device configured to measure moisture and temperature parameters inside different types of walls in a building and/or inside the space where the sensor equipment is housed, in addition to providing processing means configured to manage temperature and moisture data in real time based on sensor means that make up the sensor equipment, provide a performance profile of said variables in a specific environment, when carrying out a renovation, or treatment to avoid moisture by capillarity, and to be able to establish ways to improve the thermal insulation of a specific room and/or installation. The latter is particularly important, especially in room and building renovation operations, where problems may arise due to excess moisture in walls and facings, which would require improved thermal insulation. Another variable is knowing the effectiveness of the treatments carried out to avoid moisture by capillarity or filtration, controlling, after the treatment, the existing moisture in the walls that lose excess moisture due to capillarity or filtration.

Therefore, an object of the present invention is to provide equipment or a device configured to monitor the magnitudes of moisture and temperature in real time, both inside a specific room and inside the walls and/or architectural facings thereof, in order to validate the improvement obtained by the aforementioned renovation and insulation operations of the walls or facings and to be able to control said values over time, providing valuable information to the user, as well as to the construction companies themselves regarding the optimisation of renovation operations, use of different materials and/or work techniques.

The present invention is also particularly useful for architects when proposing a certain project, since the invention is configured as a tool capable of determining, based on the moisture data inside the walls, one system or another to alleviate a possible excess of moisture in the parameterised walls.

These objects are achieved with the equipment of claim 1. Preferred or particular embodiments of the equipment object of the present invention are described in the dependent claims.

According to the invention, there is provided a computer program as defined in claim 8.

Throughout the description and claims, the words "wall" and "architectural facing" are used interchangeably and are considered synonyms for the purposes of the present invention. Furthermore, the word "comprises" and its variants are not intended to exclude other technical features. For those skilled in the art, other objects, advantages and features of the invention may be deduced from both the invention and the practical use of the invention. The following examples and drawings are provided by way of illustration and are not intended to limit the present invention.

### Brief description of the drawings

A very brief description of a series of drawings that aid in better understanding the invention, and which are expressly related to an embodiment of said invention that is illustrated by way of a non-limiting example of the same, is provided below.
FIG. 1 shows a schematic view of the equipment of the invention

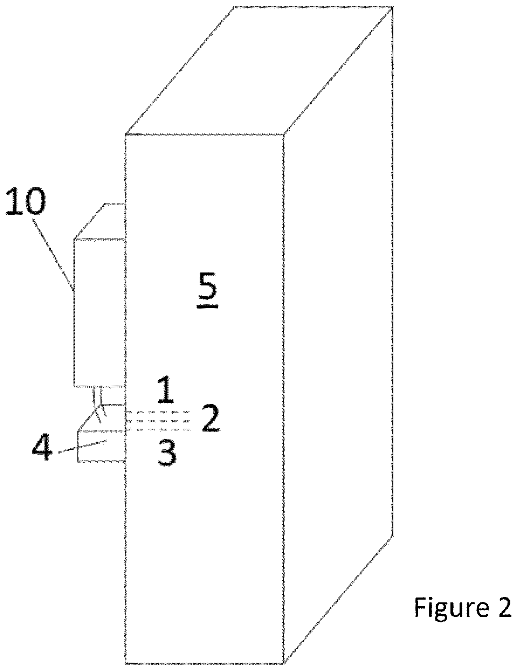

FIG. 2 shows a view of the equipment of figure 1 on a wall
FIG. 3 shows a schematic view of a second embodiment of the equipment of the invention

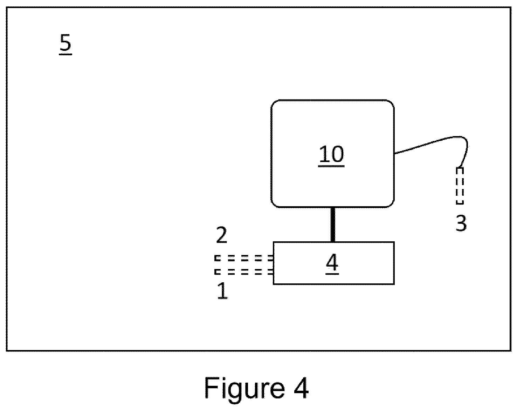

FIG. 4 shows a schematic view of another alternative embodiment of the equipment of the invention
FIG. 5 shows a schematic view of an example of a measuring apparatus not forming part of the invention as claimed
FIG. 6 shows a schematic elevation view of the example shown in figure 5

### Description of a detailed embodiment of the invention

As shown in the attached figures, the equipment of the invention is electronic equipment that consists of two essential elements: the airtight measuring body (10) and the measuring elements (1 to 4).

The airtight measuring body (10) is configured as a watertight casing, containing at least one room temperature sensor and at least one room moisture sensor, both sensors being configured, respectively, to measure the room temperature and moisture of the space in which said airtight measuring body (10) is installed.

The airtight measuring body (10), in turn, houses electronic measuring means, which will be explained in greater detail later. These electronic measuring means are configured to measure moisture and temperature in real time, both in the environment, through the aforementioned room temperature and moisture sensors, and inside a wall, for which these electronic measuring means are connected to the measuring elements (1 to 4) installed on a wall to be parameterised.

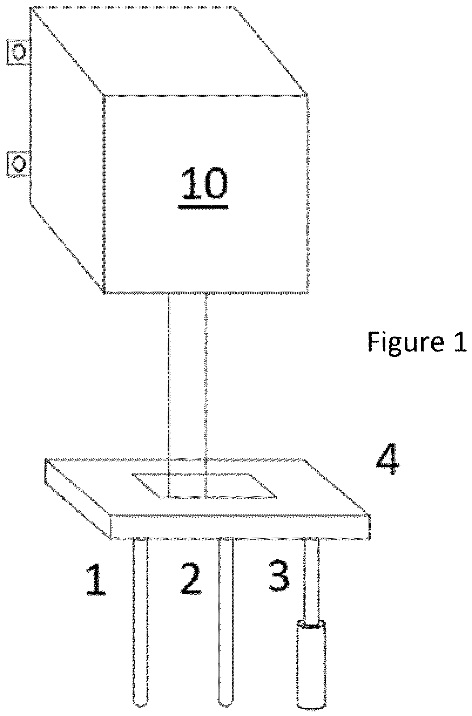

The electronic measuring means housed in the airtight measuring body (10) and the measuring elements (1, 2, 3) can be connected through one or more connection cables, as shown in the embodiment in figure 1, in which there are two connection cables.

The measuring elements (1 to 4) of the walls are made up of two internal moisture measuring rods (1,2), an internal temperature sensor (3) and a watertight support (4) configured to secure both the measuring rods (1,2) and the internal temperature sensor (3) in a specific position.

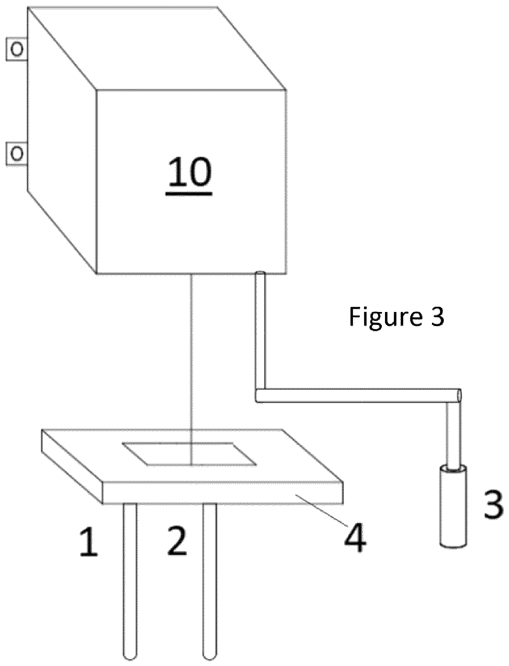

In an alternative embodiment of the invention, shown in figure 3, the internal temperature sensor (3) can be connected directly to the airtight measuring body (10), without the need to be mounted on the watertight support (4), wherein the two internal moisture measuring rods (1, 2) will be secured since said rods would require optimal airtight and watertight conditions, so that the moisture values obtained are as accurate as possible.

The electronic measuring means comprise at least one or more processors, a memory, and one or more programs, wherein the program(s) are stored in the memory and configured to be executed by the processor(s), wherein the programs include instructions for measuring the internal moisture of the walls or architectural facings by measuring the electrical resistivity that passes between the two metal internal moisture measuring rods (1,2) inserted inside the parameterisable wall (5).

Thus, given the calculation of electrical resistivity, it is possible to distinguish between the different types of walls (i.e., the different materials that make up the wall) to be monitored, and which the user of the equipment will be able to select, as will be explained in detail later.

Electrical resistivity is measured by inserting the two internal moisture measuring rods (1,2), which are metallic and cylindrical in shape, into the wall, wherein the appropriate perforations with drilling and subsequent filling with a cement mortar will have been previously carried out, through which the measuring rods (1,2) will be inserted. Once inserted into the wall, the equipment will deliver an electric discharge for a given period of time, such that the time it takes for the electric current to pass from a first rod (1) to a second rod (2) is measured, thus making it possible for the processor of the electronic equipment to establish the necessary calculation to know the percentage of moisture in the parameterised wall.

The equipment of the invention is provided with wireless communication means, preferably a SIM card or Wi-Fi connection, so that the information collected by the measuring equipment can be sent to an external server.

On the external server, a computer program or software collects said information so that the user can create installations and link each installation with the measuring equipment that the user designates. The external server is also configured so that the user can view the information from the sensors of each installed measuring equipment, the geographical location of each measuring equipment in a map application, such as, for example, Google Maps^{®}, authorise other users to see the information from the measuring equipment, and be able to attach images or documentation of the equipment or installation in a building to each installation.

On the external server, it will also be possible to select the type of wall to be parameterised, so that the calibration parameters are suited to the materials of the wall, or to define one or more internal moisture thresholds of the wall so that, upon reaching said thresholds, the server sends an alert to authorised users with said relevant information.

Finally, the equipment of the invention is electrically powered, either autonomously by batteries or connected to the electricity grid.

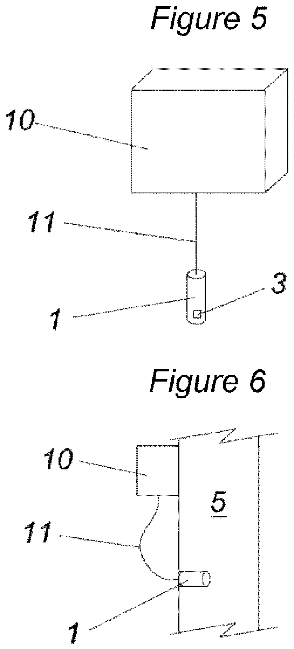

An example of a measuring equipment not forming part of the invention as claimed is shown in figures 5 and 6, where the same common parts have the same reference numbers as in the previously described embodiment. The measuring equipment is provided with a single internal moisture measuring rod (1) wherein the internal temperature sensor (3) is included.

The assembly of the equipment is as follows: The rod (1) is inserted into a hole previously made in the wall with a drill bit of a sufficient size so that the rod (1) can be housed therein. Once the internal moisture measuring rod (1) is inserted, the hole is sealed on the outside with silicone to prevent air from the outside environment from entering inside the hole, providing erroneous data. This causes the air inside the wall to reach the same moisture as the moisture present in the wall area, which means that the moisture of the air inside the wall analysed by the internal moisture measuring rod (1) inside the hole will be the same as the moisture of the wall itself, this being the same concept for detecting temperature. In this example, the connection between the electronic measuring means located in the airtight measuring body (10) and the internal moisture measuring rod (1) is through a single cable (11).

## Claims

1. Equipment for measuring moisture and temperature inside walls and at the same time measuring the moisture and temperature of the space where said equipment is housed, said equipment comprising an airtight measuring body (10) and measuring elements suitable for being installed on a parameterisable wall (5) and the airtight measuring body (10), in turn, houses electronic measuring means configured to measure moisture and temperature in real time, both in the environment and inside the parameterisable wall (5), wherein the measuring elements comprise an internal temperature sensor (3) configured to be secured in a specific internal position of the parameterisable wall (5), two internal moisture measuring rods (1,2) and a watertight support (4), wherein the watertight support (4) is configured to secure at least the two internal moisture measuring rods (1, 2) in a specific position, wherein in operation the two internal moisture measuring rods (1, 2) are inserted in two respective apertures formed in the wall and an electrically resistivity between the two internal moisture measuring rods (1, 2) is measured, the electronic measuring means comprising wireless communication means so that the information collected by the measuring equipment is sent to an external server.

2. The measuring equipment according to claim 1, wherein the measuring rods (1,2) are configured to be internally secured in a specific position of the parameterisable wall (5).

3. The measuring equipment according to any one of claims 1 to 2, wherein the temperature sensor (3) is secured to the watertight support (4) or directly to the airtight measuring body (10).

4. The measuring equipment according to any one of claims 1 to 3, wherein the electronic measuring means comprise at least:
one or more processors,
wireless communication means connected to an external server,
a memory, and
one or more programs, wherein the program(s) are stored in the memory and configured to be executed by the processor(s), wherein the programs include instructions for:
- receiving calibration parameters of the parameterisable wall (5),
- measuring room temperature and moisture,
- measuring the temperature inside the parameterisable wall (5),
- measuring the internal moisture of the parameterisable wall (5) by measuring electrical resistivity between two metal internal moisture measuring rods (1,2) inserted inside the parameterisable wall (5), and
- sending the obtained moisture and temperature measurements obtained in real time to the external server.

5. The measuring equipment according to any one of claims 1 to 4, which is electrically powered by batteries or by connection to the electricity grid.

6. The measuring equipment according to claim 1, wherein the measuring elements (1 to 4) are made up of a moisture probe that measures internal moisture (1,2).

7. The measuring equipment according to claim 1, **characterised in that** the measuring elements are made up of an internal moisture measuring rod (1) that includes the internal temperature sensor (3).

8. A computer program for communication between measuring equipment according to any of claims 1 to 7 and an external server that includes instructions which, when executed by a processor, cause the measuring equipment according to one of claims 1-7 to:
- recieve calibration parameters of the parameterisable wall (5),
- measure room temperature and moisture,
- measure the temperature inside the parameterisable wall (5),
- measure the internal moisture of the parameterisable wall (5).

9. The computer program according to claim 8, **characterised in that** it includes additional instructions for:
- Controlling the battery status level,
- Generating a map of an installation for one or more measuring equipment,
- Geographically positioning and locating the measuring equipment,
- Indicating the type of wall on which the measuring equipment is to be placed,
- Receiving a notice sent to the external server that comes from the measuring equipment when the detected moisture exceeds a pre-established threshold.

## Patentansprüche

1. Einrichtung zum Messen von Feuchtigkeit und Temperatur innerhalb von Wänden und zum gleichzeitigen Messen der Feuchtigkeit und Temperatur des Raumes, in dem die Vorrichtung untergebracht ist, wobei die Vorrichtung einen luftdichten Messkörper (10) und Messelemente umfasst, die geeignet sind, an einer parametrierbaren Wand (5) installiert zu werden, und der luftdichte Messkörper (10) seinerseits elektronische Messmittel aufnimmt, die so ausgebildet sind, dass sie Feuchtigkeit und Temperatur in Echtzeit sowohl in der Umgebung als auch innerhalb der parametrierbaren Wand (5) messen,
wobei
die Messelemente einen Innentemperatursensor (3), der dazu ausgebildet ist, in einer bestimmten Position im Inneren der parametrierbaren Wand (5) befestigt zu werden, zwei innere Feuchtigkeitsmessstäbe (1, 2) und eine wasserdichte Halterung (4) umfassen, wobei die wasserdichte Halterung (4) dazu ausgebildet ist, zumindest die beiden inneren Feuchtigkeitsmessstäbe (1, 2) in einer bestimmten Position zu befestigen, wobei im Betrieb die beiden inneren Feuchtigkeitsmessstäbe (1, 2) in zwei jeweilige in der Wand ausgebildete Öffnungen eingeführt werden und ein elektrischer Widerstand zwischen den beiden inneren Feuchtigkeitsmessstäben (1, 2) gemessen wird, wobei die elektronischen Messmittel drahtlose Kommunikationsmittel umfassen, so dass die von der Messeinrichtung gesammelten Informationen an einen externen Server gesendet werden.

2. Messeinrichtung nach Anspruch 1, **dadurch gekennzeichnet, dass** die Messstäbe (1, 2) so ausgebildet sind, dass sie in einer bestimmten Position der parametrierbaren Wand (5) innen befestigt werden.

3. Messeinrichtung nach einem der Ansprüche 1 bis 2, wobei der Temperatursensor (3) an der wasserdichten Halterung (4) oder direkt am luftdichten Messkörper (10) befestigt ist.

4. Messeinrichtung nach einem der Ansprüche 1 bis 3, wobei die elektronischen Messmittel mindestens Folgendes umfassen:
einen oder mehrere Prozessoren,
drahtlose Kommunikationsmittel, die mit einem externen Server verbunden sind,
einen Speicher, und
ein oder mehrere Programme, wobei das (die) Programm(e) in dem Speicher gespeichert und so ausgebildet ist (sind), dass sie von dem (den) Prozessor(en) ausgeführt werden, wobei die Programme Anweisungen für Folgendes enthalten:
- Empfang der Kalibrierungsparameter der parametrierbaren Wand (5),
- Messung von Raumtemperatur und Feuchtigkeit,
- Messung der Temperatur im Inneren der parametrierbaren Wand (5),
- Messung der inneren Feuchtigkeit der parametrierbaren Wand (5) durch Messung des elektrischen Widerstandes zwischen zwei metallischen inneren Feuchtigkeitsmessstäben (1, 2), die in die parametrierbare Wand (5) eingesetzt sind, und
- Übermittlung der erhaltenen Feuchtigkeits- und Temperaturmessungen in Echtzeit an den externen Server.

5. Messeinrichtung nach einem der Ansprüche 1 bis 4, das elektrisch durch Batterien oder durch Anschluss an das Stromnetz betrieben wird.

6. Messeinrichtung nach Anspruch 1, **dadurch gekennzeichnet, dass** die Messelemente (1 bis 4) aus einer Feuchtigkeitssonde bestehen, die die innere Feuchtigkeit (1, 2) misst.

7. Messeinrichtung nach Anspruch 1, **dadurch gekennzeichnet, dass** die Messelemente aus einem inneren Feuchtigkeitsmessstab (1) bestehen, der den inneren Temperatursensor (3) enthält.

8. Computerprogramm zur Kommunikation zwischen einer Messeinrichtung nach einem der Ansprüche 1 bis 7 und einem externen Server, das Anweisungen enthält, die, wenn sie von einem Prozessor ausgeführt werden, die Messeinrichtung nach einem der Ansprüche 1 bis 7 zu Folgendem veranlassen:
- Empfang von Kalibrierungsparametern der parametrierbaren Wand (5),
- Messung von Raumtemperatur und Feuchtigkeit,
- Messung der Temperatur im Inneren der parametrierbaren Wand (5),
- Messung der inneren Feuchtigkeit der parametrierbaren Wand (5).

9. Computerprogramm nach Anspruch 8, **dadurch gekennzeichnet, dass** es zusätzliche Anweisungen enthält für:
- die Kontrolle des Batteriestandes,
- das Erzeugen einer Karte einer Installation von einer oder mehrerer Messeinrichtungen,
- die geografische Positionierung und Standortbestimmung der Messeinrichtungen,
- Angabe der Art der Wand, an der die Messeinrichtung angebracht werden soll,
- Empfang einer von der Messeinrichtung an den externen Server gesendeten Meldung, wenn die festgestellte Feuchtigkeit einen vorher festgelegten Schwellenwert überschreitet.

## Revendications

1. Équipement permettant de mesurer l'humidité et la température à l'intérieur de parois et de mesurer en même temps l'humidité et la température de l'espace dans lequel ledit équipement est logé, ledit équipement comprenant un corps de mesure étanche à l'air (10) et des éléments de mesure appropriés pour être installés sur une paroi paramétrable (5) et le corps de mesure étanche à l'air (10), à son tour, loge des moyens de mesure électroniques configurés pour mesurer l'humidité et la température en temps réel, à la fois dans l'environnement et à l'intérieur de la paroi paramétrable (5),
dans lequel
les éléments de mesure comprennent un capteur de température interne (3) configuré pour être fixé dans une position interne spécifique de la paroi paramétrable (5), deux tiges de mesure d'humidité interne (1, 2) et un support étanche à l'eau (4), dans lequel le support étanche à l'eau (4) est configuré pour fixer au moins les deux tiges de mesure d'humidité interne (1, 2) dans une position spécifique, dans lequel en fonctionnement les deux tiges de mesure d'humidité interne (1, 2) sont insérées dans deux ouvertures respectives formées dans la paroi et une résistivité électrique entre les deux tiges de mesure d'humidité interne (1, 2) est mesurée, les moyens de mesure électroniques comprennent des moyens de communication sans fil de sorte que les informations collectées par l'équipement de mesure sont envoyées à un serveur externe.

2. Équipement de mesure selon la revendication 1, dans lequel les tiges de mesure (1, 2) sont configurées pour être fixées de manière interne dans une position spécifique de la paroi paramétrable (5).

3. Équipement de mesure selon l'une quelconque des revendications 1 à 2, dans lequel le capteur de température (3) est fixé au support étanche à l'eau (4) ou directement au corps de mesure étanche à l'air (10).

4. Équipement de mesure selon l'une quelconque des revendications 1 à 3, dans lequel les moyens de mesure électroniques comprennent au moins :
un ou plusieurs processeurs,
des moyens de communication sans fil connectés à un serveur externe,
une mémoire, et
un ou plusieurs programmes, dans lequel le ou les programmes sont stockés dans la mémoire et configurés pour être exécutés par le ou les processeurs, dans lequel les programmes comprennent des instructions pour :
- recevoir les paramètres d'étalonnage de la paroi paramétrable (5),
- mesurer la température et l'humidité de la pièce,
- mesurer la température à l'intérieur de la paroi paramétrable (5),
- mesurer l'humidité interne de la paroi paramétrable (5) en mesurant la résistivité électrique entre deux tiges métalliques de mesure d'humidité interne (1,2) insérées à l'intérieur de la paroi paramétrable (5), et
- envoyer au serveur externe les mesures d'humidité et de température obtenues en temps réel.

5. Équipement de mesure selon l'une quelconque des revendications 1 à 4, qui est alimenté électriquement par des batteries ou par une connexion au réseau électrique.

6. Équipement de mesure selon la revendication 1, dans lequel les éléments de mesure (1 à 4) sont constitués d'une sonde d'humidité qui mesure l'humidité interne (1, 2).

7. Équipement de mesure selon la revendication 1, **caractérisé en ce que** les éléments de mesure sont constitués d'une tige de mesure d'humidité interne (1) qui comprend le capteur de température interne (3).

8. Programme d'ordinateur pour la communication entre un équipement de mesure selon l'une quelconque des revendications 1 à 7 et un serveur externe qui comprend des instructions qui, lorsqu'elles sont exécutées par un processeur, amènent l'équipement de mesure selon l'une des revendications 1 à 7 à :
- recevoir les paramètres d'étalonnage de la paroi paramétrable (5),
- mesurer la température et l'humidité de la pièce,
- mesurer la température à l'intérieur de la paroi paramétrable (5),
- mesurer l'humidité interne de la paroi paramétrable (5).

9. Programme d'ordinateur selon la revendication 8, **caractérisé en ce qu'**il comprend des instructions supplémentaires pour:
- contrôler le niveau d'état de la batterie,
- générer une carte d'une installation pour un ou plusieurs équipements de mesure,
- positionner géographiquement et localiser l'équipement de mesure,
- indiquer le type de paroi sur lequel l'équipement de mesure doit être placé,
- recevoir une notification envoyée au serveur externe qui provient de l'équipement de mesure lorsque l'humidité détectée dépasse un seuil préétabli.
